# EUROPEAN PATENT APPLICATION

(11) **EP 2 196 450 A1**
(43) Date of publication of application: **16.06.2010**
(21) Application number: 08305906.3
(22) Date of filing: 09.12.2008
(51) Int. Cl.: C07C 51/47, C07C 66/02, C07C 69/157

(54) **Process for the preparation of pure diacetylrhein (diacerein)**

(71) Applicant: Evultis S.A., 6901 Lugano (CH)
(72) Inventor: Mazzola, Alessandro, 20149 MILANO (IT); Moiana, Silvia, 22063 CANTU-COMO (IT)
(74) Representative: Warcoin, Jacques

(57) **Abstract**

The present invention is directed to a process for producing pure Diacetylrhein (Diacerein), comprising the transformation of raw Diacerein or raw Rhein, into a water-soluble salt thereof, and then elimination of the impurities by passage through a hydrophobic resin: adsorption of the products, washing and then elution. After acidification of the pure salt of Diacerein or Rhein obtained after the passage through said hydrophobic resin, Diacerein is recovered and dried in a pure form.

The invention is also directed to pure Diacerein obtained by the process of the invention, in which the total content of impurities is between 0 and 100 ppm.

The invention is also directed to pure Diacerein obtained by the process of the invention, in which the Aloemodine content is less than 100 ppm

## Description

The present invention concerns a new process for the preparation of Diacetylrhein (Diacerein), with a high degree of purity. Such high degree of purity allows the use of the obtained Diacerein in pharmaceutical formulations for human applications.

Diacerein (chemical name: 1,8-diacetoxy-3-carboxyanthraquinone) is a molecule having anti-inflammatory activity (in particular anti-free-radicals activity) and as such can be used in the prevention and treatment of various pathological states, in particular those concerning degradation of cartilage, for example in certain forms of arthritis and osteoarthritis.

The Diacerein has the following formula:

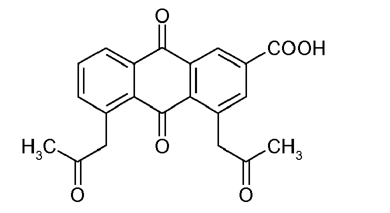

The basic products for the preparation of Diacerein are vegetable extracts deriving from plants containing the glycosilate-based anthraquinone structure, in particular from the plants Senna or Aloe. In the following, the nomenclature used will refer to products obtained from the plant Aloe, although the processes that will be described apply to all vegetable extracts, deriving for any plant that contains structures that can be transformed into anthraquinone derivatives.

Usually, the 3-carboxy-1,8-diacetylated anthraquinone product is obtained by an oxidative degradation of the sugars contained in the first-extraction products, with agents such as: chromic anhydride (DE A-4.120.989 and DE A-120.990) or FeCl₃ (WO96/30034). After this oxidation step, the corresponding anthraquinone tri-alcohol (Aloemodine) is isolated; the next step is the acetylation reaction of the phenolic and alcoholic groups present in the Aloemodine with the production of the so-called triacetate (Aloemodine Triacetate). The passage to Diacerein is achieved by transforming the methylene-alcoholic group in position 3 into the corresponding carboxylic group by means of oxidation.

As stated above, such steps belong to the state of the art.

The Diacerein thus obtained contains significant amounts of undesirable by-products, which render inappropriate its use as an active principle in pharmaceutical preparations.

Of particular importance among these - due to the various side effects such as the laxative effect - are:
- Emodine, 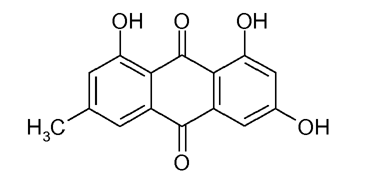
- Aloemodine and its mono-, di- and tri-acetate derivatives, 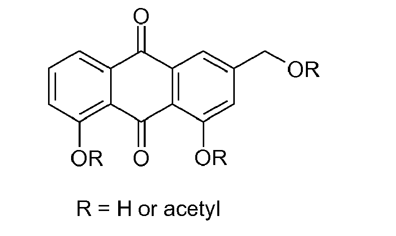 which furthermore exhibit serious genotoxic side effects.

In the present, the term "Aloemodine" means either "Aloemodine" as such or Aloemodine in admixture with the corresponding mono-, di- and/or tri-acetyl derivatives.

There are many purification processes described by various Authors. Some resort to extraction, with mixtures of organic solvents that are not particularly mixable with each other (liquid/liquid extraction), sometimes in counter-current (EP 520 414) in the presence of organic bases - such as triethylamine - which increase the solubility of the products; other Authors carry out successive precipitations of Inorganic Salts, exploiting their different solubility in various solvent mixtures (WO 2004/050601).

Depending on the process applied, the final product often has a degree of purity that is unsuitable for pharmaceutical applications, either *per* os or by injection, involving further purification steps with an obvious reduction in process yield. Particular attention must be paid to eliminating genotoxic impurities, for which Health Authorities set very precise presence limits (EMEA/CHMP/QWP/251344/2006).

Surprisingly, it has now been found a new purification process of the above-mentioned reaction raw materials (containing the said impurities), as such or in a modified form. This new purification process involves the use of appropriate resins to selectively separate Diacerein. This process results in pure Diacerein, in which impurities are not detectable by analytic means (it means less than 1 ppm of impurities). The pure Diacerein thus obtained can than be used for pharmaceutical applications in human beings, in particular both *per* os and by injection.

Thus, an object of the invention is a process for producing pure Diacetylrhein (Diacerein), comprising the following successive steps:
a. transformation of raw Diacerein or raw Rhein, which contains up to 500 ppm of Aloemodine impurities, into a water-soluble salt thereof; then
b. dissolving said salt in water;
c. adsorption of Diacerein or Rhein on a hydrophobic resin by passage of the aqueous solution obtained after step b) through a column filled with said hydrophobic resin;
d. after step c., de-adsorbing the impurities by passage of an organic solution through said column and, at the exit of the column, recovering the organic solution which contains the impurities;
e. after step d., de-adsorbing Diacerein or Rhein by passage of an hydroalcoholic solution through said column and , at the exit of the column, recovering the hydroalcoholic solution which contains Diacerein or Rhein;
f. acidification of the hydroalcoholic solution recovered after step e.;
g. if appropriate, transforming the Rhein into Diacerein;
h. recovering and drying of pure Diacerein.

The terms "pure Diacerein" means a Diacerein, in which the total impurities content is below 100 ppm, advantageously below 50 ppm, more advantageously below 10 ppm, even more advantageously below 5 ppm, even even more advantageously below 1 ppm. In particular, the Aloemodine content is below 100 ppm, advantageously below 50 ppm, more advantageously below 10 ppm, even more advantageously below 5 ppm, even even more advantageously below 1 ppm.

The raw Diacerein, used in step a., can be of a different extraction origin.

Generally speaking, the process of the present invention can be applied:
- to the raw materials obtained from Aloe and containing Diacerein with impureties (in particular emodine and Aloemodine) obtained using the methods described above (Type A Process);
- to the mixture (raw material) obtained by total deacetylation of the above described raw materials: Rhein (Type B Process). Deacetylation is performed using known methods, such as those described in WO 96/30034.

As stated above, the process can be applied both to the derivatives of any plant containing products that can potentially be transformed into glycosylate anthraquinon derivatives, in particular to derivatives of Aloe and to derivatives of Senna.

Surprisingly, the inventors have discovered that a lipophilic resin could be used to efficiently separate Diacerein or Rhein from the correlated impurities deriving from the extraction/modification of vegetable extracts. Without being bond by any theory, the inventors think that this could be explained by their difference in their degree of lipophily/hydrophily, correlated to structural differences (for example, presence of acetylated phenolic and primary alcoholic groups or not, or phenolic and acetylated groups or not, and salifiable carboxylic groups).

### Water-soluble salt:

Type A Process: The first step of the process (a.) is to salify the raw Diacerein to obtain a salt of Diacerein which is soluble in water. The water soluble salt of Diacerein is preferably an inorganic salt of Diacerein, more preferably a salt of Diacerein of an alkali metal. Such salts can be prepared by the method disclosed in EP 0 243 968. In a preferred embodiment, the salt of Diacerein is a potassium salt of Diacerein or a sodium salt of Diacerein. The solubility of potassium salt of Diacerein is around 1g/50ml of water at neutral pH (pH=7).
Type B Process: The first step of the process (a.) is to salify the raw Rhein to obtain a salt of Rhein which is soluble in water. The water soluble salt of Rhein is preferably an inorganic salt of Rhein, more preferably a salt of Rhein of an alkali metal. Such salts can also be prepared by the method disclosed in EP 0 243 968, by analogy. In a preferred embodiment, the salt of Rhein is a potassium salt of Rhein or a sodium salt of Rhein.

### Hydrophobic resin:

The resin used in the process of the invention is a hydrophobic resin.

Such an hydrophobic resin is preferably of polymer-type, wherein said polymer backbone does not comprise hydrophilic functional groups (such as OH, COOH, NH₂, ...). The polymer backbone is advantageously a hydrocarbon backbone (made from C and H atoms), which may presents hydrophobic substituents.

A hydrophobic compound does not form hydrogen bounds with the molecules of water. A hydrophobic compound is often a non-polar compound but some hydrophobic compounds can be slightly polar compounds.

As example of suitable polymer, one can cite a copolymer of styrene and divinylbenzene (PS-DVB), which may be substituted on the benzene by halogen atoms for example (in particular by brome atom).

According to a preferred embodiment, the resin is not only hydrophobic but is also porous, even more highly porous.

The porosity of the resin may for example be defined by its pore volume, which is preferably above 1 ml/g, more preferably between 1 ml/g and 2.5 ml/g.

The porous hydrophobic resin has advantageously an important specific surface area, which is preferably above 550 m²/g. In a particular embodiment, the specific area is between 550 m²/g and 1300 m²/g, more preferably between 590 m²/g and 1300 m²/g, even more preferably between 590 m²/g and 1200 m²/g.

The resin is preferably under the form of particles, whose size may range from 50 µm to 700 µm In one particular embodiment, the particle size ranges between 250 µm and 700 µm, advantageously between 250 µm and 600 µm In another particular embodiment, the particle size ranges between 50 µm and 150 µm

The resin may furthermore have a tendency to swell. In a particular embodiment, one uses a resin having a water retention capacity ranging from 40% to 70%, advantageously from 45% to 55%.

As example of appropriate commercial resin, one can refer to the DIAION® or SEPABEADS® or MCI® GEL PS-DVB resins, all produced by Mitsubishi Chemical Corporation. In particular, one can use the resin referred in the following table 1:

**Table 1: commercial resins**

| PS-DVB Adsorbents | |
|---|---|
| DIAION^{®} | HP20, HP20SS, HP21 |
| SEPABEADS^{®} | SP70,SP700,SP85,SP850,SP20SS |
| MCI^{®} GEL | CHP20A/Y/P, CHP55A/Y |

| Chemically modified PS-DVB Adsorbents | |
|---|---|
| SEPABEADS^{®} | SP207, SP207SS |

### Separation process:

The water soluble salt of raw Diacerein or Rhein is dissolved in water (step b.) and then the resulting aqueous solution is passed through a column filled with a hydrophobic resin (also called a resin bed) (step c.) prepared according to the instructions described by the Producer.

In the following, the column filled with a hydrophobic resin is also called a resin bed. The resin can also be designated by the use of the term "adsorbent".

We will then describe the best method to separate Diacerein/Rhein by passage through the column filled with the hydrophobic resin.

Said aqueous solution is percolated through resin beds, at the appropriate speed, chosen by preliminary studies. The percolation of the aqueous solution, though the resin bed, results on interactions between the molecules (Diacerein/Rhein and impurities) and the adsorbent; the interaction forces with the adsorbent being different for Diacerein/Rhein or impurities. This first step is called "adsorption step".

During the adsorption step, Diacerein/Rhein and impurities are adsorbed on the resin. The aqueous solution at the exit of the column does not contain significant amounts of Diacerein/Rhein and impurities any more.

After the adsorption step, the resin bed is washed with an appropriate organic solvent such as acetone or acetonitrile. This second step is called "washing step" (step d.). This washing step will allow de-adsorption of the impurities with the consequence that the organic solution at the exit of the column contain all the impurities but does not contain significant amounts of Diacerein/Rhein. After the washing step, the only compounds still adsorbed on the resin are Diacerein or Rhein.

Percolation in columns of the derivatives undergoing purification and washing lead to a clear separation of the fractions containing the impurities from those containing Diacerein (Type A Process) or completely deacetylated Diacerein (Rhein) (Type B Process).

After the washing step, an elution solvent is passed through the column, one or many times (at least two time). This third step is called "elution step" (step e.). This elution step will allow, by passing the elution solvent (also called eluant) over the resin to transport the Diacerein or Rhein.

In a preferred embodiment, the eluant is a mixture of water and alcohol, the alcohol being preferably methanol or ethanol. The alcohol/water ratio preferably ranges from 10%/90% to 60%/40% (the percentages being expressed in weight compared to the total weight of water and alcohol). In a preferred embodiment of the invention, at least two elution steps are performed with hydroalcohol solutions having different alcohol/water mass ratio: one start with a solution rich in water to finish with a solution rich in alcohol. In a more preferred embodiment of the invention, the first elution is performed by an ethanol or methanol/water mixture for which the mass ratio alcohol/water is about 20/80 and the last elution is performed by an ethanol or methanol/water mixture for which the mass ratio alcohol/water is about 60/40.

The number of elution steps, which depends on the nature of the resin and of the eluant, can easily be determined by the person skilled in the art with preliminary tests.

One recovers then the first fraction of eluates which contain the Diacerein or the Rhein salts and if appropriate the following fractions, which contain Diacerein or Rhein. The presence of Diacerein or Rhein in the fractions is checked by HPLC (254nm) using reference standards and related retention times. The recovered fractions, containing Diacerein or Rhein salts, are then acidified by well-known means in order to obtain the Diacerein or Rhein, in form of the base (step f.). If appropriate, the Rhein is transformed into Diacerein by well-known means (step g.). After, the pure Diacerein is recovered and dried by well-known means (step h.).

In a preferred embodiment, the fractionated collection of the eluates, performed on the basis of the signals received from the UV detection equipment, results in aqueous solutions of salts of Diacerein (or its deacetylated derivative, Rhein), from which:
a. Diacerein in form of the base can be recovered by acidification with an appropriate organic or inorganic acid (preferably a diluted strong acid such as H₂SO₄ 1M), with subsequent precipitation of the Diacerein, its filtration under vacuum, followed by washings with water, as previously described for other processes;
b. In the case of the deacetylated derivative (Rhein), after acidification with an appropriate organic or inorganic acid (preferably a diluted strong acid such as H₂SO₄ 1M), the solid residue is filtered and after complete drying is dissolved in anhydrous pyridine and acetic anhydride (adopting well-know methods previously described). Once reaction is completed, the addition of water and ice to the mixture causes the precipitation of pure Diacerein and the dissolving of the salts present. The product is recovered by filtration, preferably by filtration under vacuum, followed by washings with water.

The degree of purity of the final product is checked by HPLC on a 254 nm inverted-phase C18 column (adopting known methods described in Patents EP 0 754 173 B1 and WO 96/30034). It is practically impossible to measure the impurities content with this methodology, which means that the impurities content is below the detection threshold (below 1 ppm).

The yield of Processes A and B are on average around 90-95% if referred to the raw Diacerein or raw Rhein content, in form of the base, which may contain no more than 500 ppm of impurities, particularly Aloemodine.

The following examples, which in no way limit the scope of the invention, illustrate the preferred embodiments of the process.

### EXAMPLE 1

5g of raw Diacerein containing 250 ppm of Aloemodine derivatives are suspended in 75ml of acetone and 5ml of water and, under magnetic agitation, 2.5ml of triethylamine are added. The solution thus obtained is treated at 18°C with 18ml of a 1M solution of potassium Ethyl Hexanoate in isobutanol diluted with 26ml of acetone. Salification agent is added over a period of 2 hours. A precipitate is formed which is filtered, washed with acetone and dried under vacuum at 40°C for one night.

5g of Potassium Salt of Diacerein are obtained.

2g of this product are dissolved in 200ml of water (final pH of the solution 6.2). This solution, after filtration under vacuum, is percolated through a 7.5cm-diameter 10cm-high column, packed with 180g of SEPABEADS® SP207® (Mitsubishi).

The typical characteristics of SEPABEADS® SP207® are given in the table 2 below:

**Table 2: characteristics of SEPABEADS® SP207®**

| | |
|---|---|
| Water retention % | 45 -55 |
| Particle size 205 µm | 250 - 600 |
| Specific surface area (m²/g) | 590 |
| Specific gravity | 1.18 |
| Pore volume (ml/g) | 1.1 |
| Average pore radius (Å) | 120 |

Washing with a volume corresponding to the volume of the column of acetone and then elution with a ethanol/water mixture are performed until the complete elution of the Diacerein. 4 elution steps are performed: the two first elutions are performed by using ethanol/water mixture 20%/80% and the two last elutions are performed by using ethanol/water mixture 60%/40%.

The fraction containing the product is then brought to pH 4.5-5 with 10% sulphuric acid. The suspension is cooled to 5-10°C, the precipitate recovered by filtration under vacuum, washed with cold water and dried under vacuum.

1.3g of Diacerein are obtained which on HPLC analysis are found to be free of impurities.

### EXAMPLE 2

5g of raw Diacerein containing about 300 ppm of Aloemodine derivatives is dissolved in 40ml of methanol and, under magnetic agitation, 40ml of water and 5g of KOH are added. In the presence of a condenser, heating to 60-65°C is performed for 30 minutes; after this period, 35ml of 6N HCL are added; dilution with about 35ml of water is performed and the solution is boiled for about 30 minutes. After cooling, the suspension is filtered under vacuum, the residue washed with water and dried under vacuum at constant weight.

4.5g of Rhein are thus obtained.

2g of Rhein thus obtained are transformed into the corresponding potassium salt as described for the Diacerein in Example 1.

2g of Potassium Salt of Rhein are dissolved in 200ml of water (final pH of the solution 6.2). This solution, after filtration under vacuum, is percolated through a 7.5cm-diameter 10cm-high column, packed with 180g of Diaion SP207® (Mitsubishi).

Washing with a volume corresponding to the volume of the column of acetone and then elution with a water/ethanol mixture are performed until the complete elution of the Rhein. 4 elution steps are performed: the two first elutions are performed by using ethanol/water mixture 20%/80% and the two last elutions are performed by using ethanol/water mixture 60%/40%.

The fraction containing the Rhein is then brought to pH 4.5-5 with 10% sulphuric acid. The suspension is cooled to 5-10°C, the precipitate recovered by filtration under vacuum, washed with cold water and dried under vacuum.

The precipitate, after drying, is acetylated using pyridine and acetic anhydride in a ratio of 1:1 (alternatively, other conventional acetylating agents may be used).

After drying under vacuum, 1.5g of Diacerein are obtained which under HPLC analysis are shown to be free of impurities.

The yield of the process, from the Potassium Salt of Rhein, is 87%.

## Claims

1. A process for producing pure Diacetylrhein (Diacerein), comprising the following successive steps:
a. transformation of raw Diacerein or raw Rhein, which contains up to 500 ppm of Aloemodine impurities, into a water-soluble salt thereof; then
b. dissolving said salt in water;
c. adsorption of Diacerein or Rhein on a hydrophobic resin by passage of the aqueous solution obtained after step b) through a column filled with said hydrophobic resin;
d. after step c., de-adsorbing the impurities by passage of an organic solution through said column and, at the exit of the column, recovering the organic solution which contains the impurities;
e. after step d., de-adsorbing Diacerein or Rhein by passage of an hydroalcoholic solution through said column and, at the exit of the column, recovering the hydroalcoholic solution which contains Diacerein or Rhein;
f. acidification of the hydroalcoholic solution recovered after step e.;
g. if appropriate, transforming the Rhein into Diacerein;
h. recovering and drying of pure Diacerein.

2. The process according to claim 1, wherein in step a. the water soluble salt is an inorganic salt, preferably a salt of an alkali metal.

3. The process according to claim 2, wherein in step a. raw Diacerein or raw Rhein is transformed into potassium salt or sodium salt thereof.

4. The process according to anyone of the preceding claims, wherein in step c. the hydrophobic resin is a copolymer of styrene and divinylbenzene (PS-DVB), which may be substituted on the benzene by halogen atoms.

5. The process according to anyone of the preceding claims, wherein in step c. the hydrophobic resin is furthermore porous.

6. The process according to anyone of the preceding claims, wherein step e. is repeated at least two times and one collects each hydroalcoholic solution exiting the column.

7. The process according to anyone of the preceding claims, wherein step e. is performed with the use of water/ethanol or water/methanol mixtures as eluant.

8. The process according to claim 7, wherein the alcohol/water mass ratio ranges from 10%/90% to 60%/40% (w/w).

9. Pure Diacerein obtained by the process according to anyone of claims 1 to 8, in which the total content of impurities is between 0 and 100 ppm.

10. Pure Diacerein obtained by the process according to anyone of claims 1 to 8, in which the Aloemodine content is less than 100 ppm.
